# EUROPÄISCHE PATENTSCHRIFT

(11) **EP 1 286 161 B1**
(45) Veröffentlichungstag und Bekanntmachung des Hinweises auf die Patenterteilung: **14.05.2008**
(21) Anmeldenummer: 02016524.7
(22) Anmeldetag: 24.07.2002
(51) Int. Cl.: G01N 33/28

(54) **Einrichtung zur Ermittlung der Qualität von Motorenöl und eine Motor- und/oder Abgastesteinrichtung sowie ein Verfahren zum Betreiben derselben**
Device for assessing motor oil quality and an apparatus and method for testing engine or exhaust-gas using the same
Dispositif pour évaluer la qualité d'huile du moteur et dispositif et méthode pour examiner le moteur ou les gaz d'échappement

(30) Priorität: 21.08.2001 DE 10140917
(43) Veröffentlichungstag der Anmeldung: 26.02.2003
(73) Patentinhaber: ROBERT BOSCH GMBH, 70442 Stuttgart (DE)
(72) Erfinder: Mai, Hans, 73035 Goeppingen (DE)
(74) Vertreter: Gleiss & Grosse

(56) Entgegenhaltungen:
- WO-A-00/45145
- GB-A- 2 342 445
- US-A- 5 929 754

## Beschreibung

Die Erfindung betrifft eine Einrichtung zur Ermittlung wenigstens zweier Parameter eines Öls in einem Motor, insbesondere Brennkraftmaschine eines Fahrzeugs, gemäß Oberbegriff des Anspruchs 1.

### Stand der Technik

Einrichtungen der hier angesprochenen Art sind bekannt. Sie werden üblicherweise in einer Kraftfahrzeug-Werkstatt zur Motor- und Emissionsprüfung von Fahrzeugmotoren eingesetzt. Die Einrichtung umfasst einen Sensor, beispielsweise ein Halbleiter- oder Thermoelement, mit dem die Temperatur des Motorenöls ermittelt wird.

Es ist ferner bekannt, das in einem Motor eines Fahrzeugs befindlichen Öl zu überprüfen, ob es die daran gestellten Anforderungen noch erfüllt, um einen vorzeitigen, noch unnötigen Ölwechsel zu vermeiden. Hierzu wird bei einem bekannten Verfahren dem Motorenöl eine Probe entnommen, was in der Regel in einer Fahrzeugwerkstatt erfolgt. Die Probe wird dann in ein Labor geschickt, in dem es analysiert wird. Nachteilig hierbei ist, dass das Analyseergebnis in der Regel erst nach einem Tag vorliegt.

Es ist ferner bekannt, in den Motor eines Fahrzeugs einen Sensor zur Überprüfung der Schmierstoffqualität des sich im Motor des Fahrzeugs befindlichen Öls zu integrieren. Da die Kosten hierfür relativ hoch sind, sind derartige Sensoren bisher nur für Fahrzeuge der Oberklasse vorgesehen.

US 5,929,754 offenbart einen Multifunktionssensor einer Motortesteinrichtung eines Automobils, der an einen Ölbehälter einer Brennkraftmaschine des Automobils festgeschraubt ist und als Teil eines Kontrollsystems des Automobils arbeitet.

### Vorteile der Erfindung

Die erfindungsgemäße Einrichtung mit den in Anspruch 1 genannten Merkmalen bietet demgegenüber den Vorteil, dass mit dem als Multifunktionssensor ausgebildeten Sensor bei praktisch allen Fahrzeugen außer der Öltemperatur auch das Öl kontrolliert werden kann. Hierzu wird wenigstens ein die Ölqualität definierender Hilfsparameter ermittelt, beispielsweise die Viskosität (η), die elektrische Leitfähigkeit, die Dichte (ρ) oder die Dielektrizitätszahl (ε). Durch die Ölprüfung können die Betriebskosten der Fahrzeuge verringert werden, da die starren, vom jeweiligen Fahrzeughersteller vorgegebenen Ölwechselintervalle durch vorzugsweise regelmäßige Kontrollen verlängert werden können. Dadurch entsteht weniger Sondermüll, so dass die Umweltbelastung reduziert ist und die Ressourcen geschont werden.

Besonders vorteilhaft ist, wenn die Einrichtung in einer Werkstatt für Fahrzeuge stationär angeordnet ist. Es muss also nicht jedes Fahrzeug mit dem Multifunktionssensor ausgestattet werden. Dadurch ist eine regelmäßige und preiswerte Ölkontrolle ohne großen Aufwand möglich, was beispielsweise bei einer routinemäßigen Inspektion des Fahrzeugs, zu dem dieses ohnehin in die Werkstatt gebracht wird, erfolgen kann. Das Auswerteergebnis liegt zeitnah vor, so dass bei festgestellter Unterschreitung einer geforderten Ölmindestqualität das Öl gegebenenfalls gleich ausgewechselt werden kann.

Nach einer Weiterbildung der Erfindung ist vorgesehen, dass die Einrichtung in eine Motor- und/oder Abgastesteinrichtung für eine Werkstatt integriert ist. Dies ermöglicht bei der Abgasuntersuchung, wo grundsätzlich die Öltemperatur gemessen werden muss, auch eine Überprüfung des Motorenöls hinsichtlich seiner Qualität.

Es wird ein Ausführungsbeispiel der Einrichtung bevorzugt, bei dem eine Auswerteeinrichtung vorgesehen ist, mittels derer der mindestens eine ermittelte Hilfsparameter zur Bestimmung der Qualität des sich im Motor befindlichen Öls ausgewertet wird. In bevorzugter Ausführungsform ist die Auswerteeinrichtung in eine Motor- und/oder Abgastesteinrichtung integriert. Das Diagnoseergebnis kann beispielsweise von der Motor- und/oder Abgastesteinrichtung angezeigt werden. Es ist ohne weiteres möglich, dass die Einrichtung eine eigenständige, transportable Einheit ist, die gegebenenfalls an eine Motor- und/oder Abgastesteinrichtung angeschlossen werden kann.

Weiterhin wird ein Ausführungsbeispiel der Einrichtung bevorzugt, das sich dadurch auszeichnet, dass der Multifunktionssensor in das Öl eintauchbar ist. Dies kann beispielsweise über einen Ölmessstabstutzen erfolgen, den üblicherweise alle Kraftfahrzeuge besitzen. Dadurch ist der Multifunktionssensor praktisch bei allen Fahrzeugtypen universell einsetzbar.

Weitere vorteilhafte Ausführungsformen der Einrichtung ergeben sich aus Kombinationen der in den Unteransprüchen genannten Merkmale.

Der Gegenstand der Erfindung betrifft auch eine Motor- und/oder Abgastesteinrichtung für Motoren, insbesondere Brennkraftmaschinen von Fahrzeugen, gemäß Anspruch 9, und ein Verfahren zum Betreiben einer Motor- und/oder Abgastesteinrichtung für Motoren, insbesondere Fahrzeugmotoren, gemäß Anspruch 10.

### Zeichnungen

Die Erfindung wird nachfolgend in einem Ausführungsbeispiel anhand der Zeichnungen näher erläutert. Es zeigen:
- Figur 1: ein Ausführungsbeispiel einer mobilen Einrichtung zur Ermittlung von Ölparametern;
- Figur 2: einen mit gestrichelter Linie eingekreisten Ausschnitt der in Figur 1 dargestellten Einrichtung in vergrößertem Maßstab, nämlich einen Multifunktionssensor; und
- Figur 3: einen Längsschnitt durch den Multifunktionssensor gemäß Figur 2.

### Beschreibung des Ausführungsbeispiels

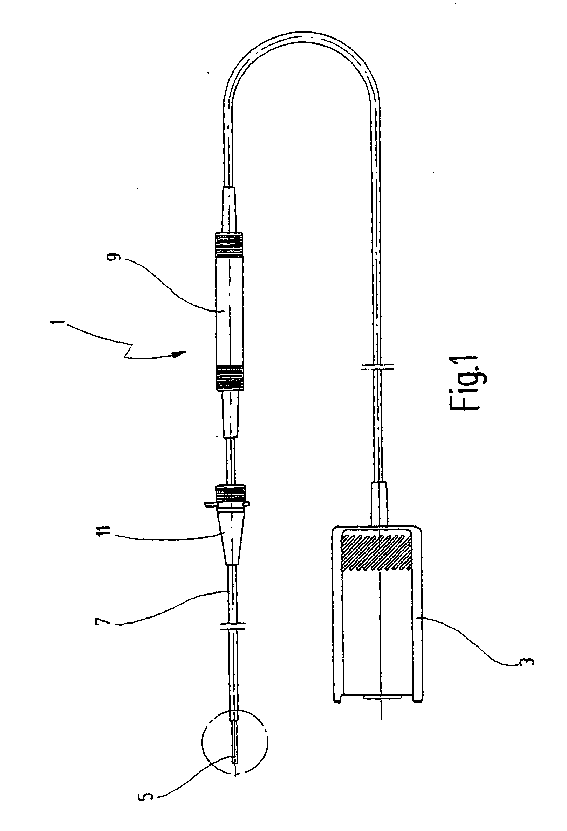

Figur 1 zeigt ein Ausführungsbeispiel einer Einrichtung 1 zur Ermittlung von Parametern eines in einem Motor, beispielsweise einer Brennkraftmaschine eines Fahrzeugs befindlichen Öls. Die Einrichtung 1 umfasst ein Gehäuse 3, in dem sich eine nicht dargestellte Auswerteeinrichtung, vorzugsweise eine Auswerteelektronik befindet, und einen Multifunktionssensor 5, mittels dessen unter anderem die Öltemperatur und andere, für die Ermittlung der Ölqualität erforderliche Größen erfasst werden können.

Der Multifunktionssensor 5 ist am Ende einer langen, in Figur 1 verkürzt dargestellten biegsamen Welle 7 angeordnet, die als Verbindungselement zwischen dem Multifunktionssensor 5 und einem Handgriff 9 dient. Dieser wird von der Welle 7 durchsetzt. Die Welle 7 ist eine Hohlwelle, in der mindestens eine Verbindungsleitung zur Kopplung des Multifunktionssensors 5 mit der im Gehäuse 3 befindlichen Auswertelektronik untergebracht ist. Wie in Figur 1 gezeigt, ist die Welle 7 über einen Anschlussstecker mit der Auswerteelektronik verbunden. Der Handgriff 9 weist vorzugsweise einen nicht dargestellten Einbauraum für Elektronikkomponenten auf.

Der Multifunktionssensor 5 wird über einen Ölmessstabstutzen des Motors eingeführt in das sich im Allgemeinen in einer Ölwanne gesammelte Ölbad eingetaucht. Zum Abdichten des Ölmessstabstutzens ist ein arretierbarer Verschlussstopfen 11 vorgesehen, der zwischen dem Multifunktionssensor 5 und dem Handgriff 9 angeordnet ist und von der Welle 7 durchdrungen wird.

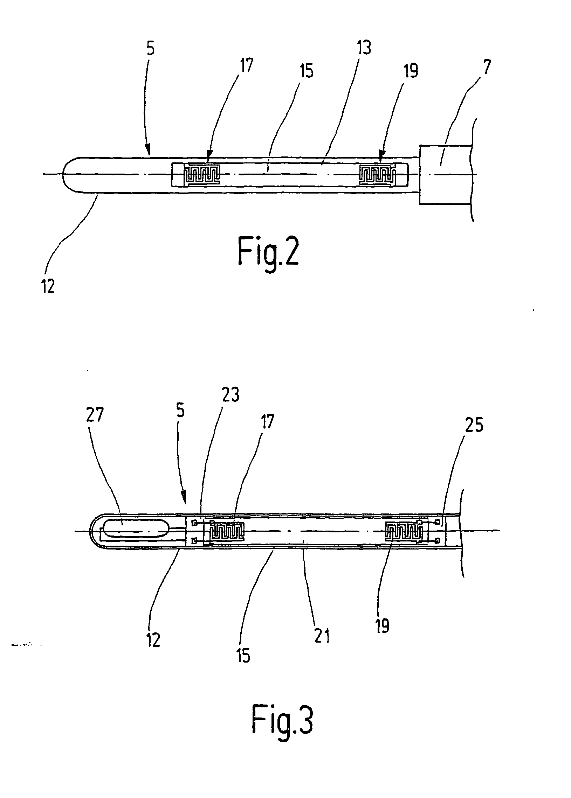

In Figur 2 ist der Multifunktionssensor 5 in stark vergrößertem Maßstab dargestellt. Der Multifunktionssensor 5 weist ein Schutzrohr 12 mit einem länglichen Durchbruch 13, durch den das Öl in einen Innenraumbereich 15 des Schutzrohrs 12 gelangt.

Wie aus Figur 3 ersichtlich, die einen Längsschnitt durch den Multifunktionssensor 5 zeigt, sind im Innenraumbereich 15 ein Oberflächenwellensensor 17 (Interdigital-Transducer) zur Messung der Zähflüssigkeit und ein Interdigital-Kondensator 19 zur Messung der Leitfähigkeit und der Dielektrizitätszahl des Öls angeordnet, die auf einem Quarz 21 angebracht sind. Der Oberflächenwellensensor 17 ist mit einem ersten Chipträger 23 und der Interdigital-Kondensator 19 mit einem zweiten Chipträger 25 elektrisch verbunden. Ferner ist ein Temperaturfühler 27, beispielsweise ein Halbleiter- oder ein Thermoelement, zur Ermittlung der Öltemperatur vorgesehen, der in einem vom Innenraumbereich 15 getrennten Abschnitt des Schutzrohrs 12 angeordnet ist. Der Aufbau und die Funktion des Interdigital-Kondensators 19, des Oberflächenwellensensors 17 sowie des Temperaturfühlers 27 sind bekannt, so dass hier nicht näher darauf eingegangen wird.

Zur Ermittlung der Öltemperatur und der Ölqualität muss lediglich der Multifunktionssensor 5 in das Öl eingetaucht werden. Die Auswertung der mittels des Interdigital-Kondensators 19 und des Oberflächenwellensensors 17 ermittelten Hilfsparameter hinsichtlich der Qualität des Öls erfolgt durch die Auswerteeinrichtung. Das Auswertungsergebnis, das sehr schnell vorliegt, und die Öltemperatur kann beispielsweise an einer, in den Figuren nicht dargestellten Anzeige abgelesen werden.

Aufgrund der einfachen Handhabung der Einrichtung 1 kann diese beispielsweise in einer Werkstatt für Fahrzeuge ohne weiteres eingesetzt werden. Die Einrichtung 1 ermöglicht eine schnelle und preiswerte Kontrolle des Öls, ohne dass dazu eine Probe entnommen werden muss.

Bei einem anderen, nicht dargestellten Ausführungsbeispiel ist die Einrichtung 1, insbesondere die Auswerteeinrichtung, in eine Motor- und/oder Abgastesteinrichtung für Motoren integriert. Da beispielsweise bei einer Abgasuntersuchung ohnehin die Öltemperatur gemessen werden muss, kann mittels des mobilen Multifunktionssensors 5 zeitgleich auch die Ölkontrolle durchgeführt werden. Alternativ kann vorgesehen sein, dass die Einrichtung 1 auch an die Motor- und/oder Abgastesteinrichtung angeschlossen beziehungsweise anschließbar ist.

## Patentansprüche

1. Motor- und/oder Abgastesteinrichtung, insbesondere für Brennkraftmaschinen von Fahrzeugen, mit einer Vorrichtung zur Motor- und/oder Emissionsprüfung und einer Einrichtung, die die Qualität des Motoröls überprüft, wobei die Einrichtung (1) wenigstens zwei Parameter des Motoröls ermittelt und hierzu mindestens einen Sensor (17; 19; 27) aufweist, der als Multifunktionssensor (5) zur Bestimmung der Öltemperatur und wenigstens eines die Qualität definierenden Hilfsparameters ausgebildet ist, und mit einer Auswerteeinrichtung, mittels derer der mindestens eine ermittelte Hilfsparameter zur Bestimmung der Qualität des sich im Motor befindlichen Öls ausgewertet wird, **dadurch gekennzeichnet, dass** mittels der Auswerteeinrichtung ein Auswertungsergebnis visuell bereitgestellt wird, und dass der Multifunktionssensor (5) über einen Ölmessstabstutzen des Motors in das Öl eintauchbar ist, wobei der Multifunktionssensor (5) am Ende einer langen, biegsamen Welle (7) angeordnet ist, die als Verbindungselement zwischen dem Multifunktionssensor (5) und einem Handgriff (9) dient, welcher von der Welle (7), die als Hohlwelle ausgebildet ist, durchsetzt wird.

2. Einrichtung nach Anspruch 1, **dadurch gekennzeichnet, dass** mittels des Multifunktionssensors (5) die Viskosität (η), die elektrische Leitfähigkeit, die Dichte (ρ) und/oder die Dielektrizitätszahl (ε) ermittelbar ist.

3. Einrichtung nach einem der vorhergehenden Ansprüche, **dadurch gekennzeichnet, dass** der Multifunktionssensor (5) an die Motor- und/oder Abgastesteinrichtung angeschlossen oder anschließbar ist.

4. Einrichtung nach einem der vorhergehenden Ansprüche, **dadurch gekennzeichnet, dass** die Einrichtung (1) in einer Werkstatt für Fahrzeuge stationär angeordnet ist.

## Claims

1. Engine and/or exhaust gas testing device, in particular for internal combustion engines of vehicles, having an apparatus for testing the engine and/or emissions, and a device which checks the quality of the engine oil, wherein the device (1) determines at least two parameters of the engine oil and for this purpose has at least one sensor (17; 19; 27) which is designed as a multifunction sensor (5) for determining the temperature of the oil and at least one auxiliary parameter which defines the quality, and having an evaluation device by means of which the at least one auxiliary parameter which is determined is evaluated in order to determine the quality of the oil located in the engine, **characterized in that** an evaluation result is made available visually by means of the evaluation device, and **in that** the multifunction sensor (5) can be dipped into the oil by means of an oil measuring connector of the engine, wherein the multifunction sensor (5) is arranged at the end of a long flexible shaft (7) which serves as a connecting element between the multifunction sensor (5) and a handle (9) which is penetrated by the shaft (7) which is embodied as a hollow shaft.

2. Device according to Claim 1, **characterized in that** the viscosity (η), the electrical conductivity, the density (ρ) and/or the dielectric constant (ε) can be determined by means of the multifunction sensor (5).

3. Device according to one of the preceding claims, **characterized in that** the multifunction sensor (5) is connected or can be connected to the engine and/or exhaust gas testing device.

4. Device according to one of the preceding claims, **characterized in that** the device (1) is arranged in a fixed fashion in a workshop for vehicles.

## Revendications

1. Installation pour tester un moteur et/ou les gaz d'échappement, notamment pour des moteurs à combustion interne de véhicules automobiles, comprenant un dispositif de contrôle du moteur et/ou des émissions ainsi qu'une installation vérifiant la qualité de l'huile du moteur,
l'installation (1) déterminant au moins deux paramètres de l'huile du moteur et elle comporte à cet effet au moins un capteur (17, 19 ; 27) réalisé comme capteur multifonctions (5) pour déterminer la température de l'huile et au moins l'un des paramètres auxiliaires définissant la qualité ainsi qu'une installation d'exploitation pour exploiter au moins un paramètre auxiliaire déterminé pour définir la qualité de l'huile contenue dans le moteur,
**caractérisée en ce qu'**
à l'aide de l'installation d'exploitation on fournit visuellement un résultat d'exploitation et
le capteur multifonctions (5) peut être plongé dans l'huile par l'intermédiaire d'un ajutage en forme de tige de mesure d'huile du moteur, le capteur multifonctions (5) étant installé à l'extrémité d'un long axe souple (7) servant d'élément de liaison entre le capteur multifonctions (5) et une poignée (9) traversée par l'arbre (7) en forme d'arbre creux.

2. Installation selon la revendication 1,
**caractérisée en ce qu'**
à l'aide du capteur multifonctions (5) on détermine la viscosité (ν), la conductivité électrique, la densité (ρ) et/ou le coefficient diélectrique (ε).

3. Installation selon l'une des revendications précédentes,
**caractérisée en ce que**
le capteur multifonctions (5) est raccordé ou peut être raccordé à l'installation de détection du moteur et/ou des gaz d'échappement.

4. Installation selon l'une des revendications précédentes,
**caractérisée en ce que**
l'installation (1) est située de manière fixe dans un atelier de réparation automobile.
